# EUROPEAN PATENT SPECIFICATION

(11) **EP 3 196 315 B1**
(45) Date of publication and mention of the grant of the patent: **05.02.2020**
(21) Application number: 16466002.9
(22) Date of filing: 19.01.2016
(51) Int. Cl.: C12N 11/14, C12Q 1/46

(54) **SPHERICAL PELLETS, MANUFACTURING PROCESS OF SUCH PELLETS, USE, AND A DETECTION TUBE COMPRISING SUCH PELLETS**
KUGELFÖRMIGE PELLETS, HERSTELLUNGSVERFAHREN SOLCHER PELLETS, VERWENDUNG UND EINE DETEKTIONSRÖHRE MIT SOLCHEN PELLETS
PASTILLES SPHÉRIQUES, PROCESSUS DE FABRICATION ASSOCIÉ, UTILISATION ET TUBE DE DÉTECTION COMPRENANT DE TELLES PASTILLES

(43) Date of publication of application: 26.07.2017
(73) Proprietor: ORITEST spol. s r.o., 150 00 Praha 5 (CZ); Veterinární a farmaceutická univerzita Brno, 612 42 Brno (CZ)
(72) Inventor: Pitschmann, Vladimír, 1282 Praha 5 (CZ); Matejovský, Lukás, Hostivice (CZ); Vetchý, David, Hvozdec (CZ); Franc, Ales, Brno (CZ)
(74) Representative: Pavlica, Tomas

(56) References cited:
- EP-A1- 0 083 582
- WO-A1-94/05808
- CZ-A3- 9 301 179
- SU-A1- 707 923
- US-A1- 2010 330 169
- VLADIMÍR PITSCHMANN ET AL: "Enzymatic Determination of Anticholinesterases Using a Composite Carrier", ANALYTICAL LETTERS, 7 March 2016 (2016-03-07), XP055266710, US ISSN: 0003-2719, DOI: 10.1080/00032719.2016.1151889
- VETCHÝ DAVID ET AL: "Preparation and evaluation of carriers for detection of cholinesterase inhibitors", NEUROENDOCRINOLOGY LETTERS, MAGHIRA & MAAS PUBLICATIONS, SE, vol. 36, no. Suppl. 1, 15 October 2015 (2015-10-15), pages 95-99, XP009189651, ISSN: 0172-780X
- ONDREJ HOLAS ET AL: "The progress in the cholinesterase quantification methods", EXPERT OPINION ON DRUG DISCOVERY, vol. 7, no. 12, 26 September 2012 (2012-09-26), pages 1207-1223, XP055267484, London, GB ISSN: 1746-0441, DOI: 10.1517/17460441.2012.729037
- VETCHÝ DAVID ET AL: "[Methods of pharmaceutical technology in preparation of pellets for detection of acetylcholinesterase inhibitors].", CESKÁ A SLOVENSKÁ FARMACIE : CASOPIS CESKÉ FARMACEUTICKÉ SPOLECNOSTI A SLOVENSKÉ FARMACEUTICKÉ SPOLECNOSTI OCT 2012, vol. 61, no. 5, 4 October 2012 (2012-10-04), - 18 October 2012 (2012-10-18), pages 234-239, XP009189710, ISSN: 1210-7816

## Description

### Field of the invention

The present invention relates to the spherical pellets, containing an immobilized enzyme based on cholinesterase in activity of 1 to 100 nkat.g⁻¹, particularly designed for the detection of cholinesterase inhibitors by enzymatic hydrolysis of substrate acetylthiocholine and/or butyrylthiocholine producing the corresponding thiol, which is indicated by a color change in a redox indicator or by enzymatic hydrolysis of a chromogenic substrate. The present invention further pertains to the method of preparing such pellets by extrusion-spheronization or rotogranulation, utilization of such pellets for the detection of cholinesterase inhibitors, and a detection tube comprising these spherical pellets and specifically intended for the detection of nerve agents in the environment.

### Background of the invention

The immobilized enzymes are usually proteases physically enclosed in a limited area without losing their specific enzymatic activity. They can be used repeatedly and/or continuously. Immobilization ensures a sufficient contact of the reactants with the enzyme, wherein the reactant is generally present in the phase which passes through the solid phase containing the immobilized enzyme. The reactant can eventually be contained in the gaseous phase, which is immiscible with water. The solid phase should have a large specific surface, it should be water insoluble, hydrophilic, and chemically, mechanically, and thermally stable. Moreover, it should have a suitable shape and a size of the particles and it should be resistant against microbial activity and allow repeated and/or continuous utilization. Several methods are currently used to immobilize the enzymes.

### Methods of immobilization

The enzymes based on cholinesterase have been known in the immobilized state for years. A number of methods is used for their immobilization, just as in the case of other proteases. This includes particularly sorption to an insoluble carrier, binding to ion exchangers, incorporation into membranes and films, incorporation into gels and foams, crosslinked aggregates, and immobilization using nanostructures, covalent binding, or antibodies. Generally, it can be stated that substrates and techniques used for enzyme immobilization are widely employed in the biochemical industry. A number of protected methods is presented in the patent and scientific literature. These methods sometimes cannot be clearly discerned as they combine several different principles. All of the methods appear in the different detection systems, either individually or combined.
The following taxonomy is listed for better lucidity.

### a) Sorption to an insoluble carrier

This is the oldest of the immobilization methods. Different binding forces can act between the sorbent and the enzyme, e.g. cohesion, adhesion, hydrogen bond, Van der Waals forces, etc.

U.S. patent No. 3,049,411 (1962) describes immobilization of cholinesterase by filter paper or silica gel. The sorbent was impregnated by a 4% solution of the enzyme in bovine albumin, gelatin, or other stabilizing protein and the mixture was dried. In detection of the potential contamination, the air is drawn through the wetted disc of filter paper previously treated with the enzyme solution. The developing reagent containing indoxyl acetate was then applied to the disc. The active cholinesterase hydrolyzed indoxyl acetate to indoxyl which was then oxidized by air to indigo. When stored in a dry atmosphere, the activity of the immobilized enzyme was retained for extended time periods even at higher temperatures.

U.S. patent No. 4,241,180 (1980) also discloses filter paper as a carrier for cholinesterase. 2-mercaptoethanol was used for impregnation instead of albumin and similar results were obtained.

U. S. patent No. 3,666,627 (1971) introduces an innovative step of impregnation. The enzyme was adsorbed to the carrier of porous silica glass in the presence of the substrate. The active site of the enzyme was therefore protected and the enzyme had higher activity and stability than the free unbound enzyme.

U. S. patent No. 3,802,997 (1972) claims an analogical method of enzyme immobilization supplemented by spray drying or lyophilization of the product. An easier method for enzyme paper preparation is proposed in U. S. patent No. 4,120,754 (1978) which discloses immobilization of butyrylcholinesterase on filter paper by impregnation with solution of enzyme in buffer and Tergitol 15-S-12. The enzyme paper was used for detection of organophosphate nerve agents.

Cholinesterases were adsorbed on bleached cotton fabric and granulated cellulose in patents Nos. CZ288576 (1994) and CZ285242 (1995), respectively.

U. S. patent No. 2,008,160,556 (2008) protects a modification of filter paper for immobilization of acetylcholinesterase intended for preparation of strips for detection of acetylcholinesterase inhibitors. At first, filter paper was impregnated by calcium caseinate. After drying, a mixed solution of the enzyme, trehalose and Ellman's reagent was applied on the impregnated paper. Another materials can be used as the insoluble carriers, e.g. thin glass capillary tubes (J. Environmental Anal. Chem. 1980, 8(4): 277-282), polystyrene film (Toxicol. Appl. Pharmacol., 1984, 73(1): 105-109), or the surface of an electrode (NATO ASI Series, 1988 226: 187-194).

The spherical pellets consisting of microcrystalline cellulose, with or without incorporated aluminum oxide and colloidal silica, also serve as an insoluble carrier for immobilization of acetylcholinesterase (Čes. slov. farm., 2012; 61(5), 234-239).

### b) Binding to ion exchangers

U. S. patent No. 4,324,858 (1982) claims the process of immobilization and stabilization of cholinesterase on a cellulose base. The sorbent consists of a DEAE or ECTEOLA ion exchange paper with basic groups covalently bond to cellulose. Plasma of an electric eel (Electrophorus electricus) was used as a donor of cholinesterase.

Patent No. EP0176585B1 discloses a ion exchange paper containing diethylaminoethyl groups for impregnation of cholinesterase from plaice. The optimal results were reached when the paper was equilibrated with a sodium chloride solution, dried, impregnated by a buffered solution comprising cholinesterase, saccharose and dextran as stabilizing agents, and Tween 80 as a wetting agent, and then dried. The enzyme papers maintained a satisfactory storage stability over a long storage time when stored in the welded polypropylene bags under high temperature. The presence of saccharose was required to achieve good stability results.

### c) Binding to magnetic particles

The magnetic particles with immobilized cholinesterase were used to determine cholinesterase inhibitors by flow injection analysis (FIA). The immobilized protein was released after an electric impulse. Besides the magnetic particles, a porous glass (J. Chromatogr. A., 1991, 539(1): 47-54) or methacrylates can be used for FIA (Anal. Chim. Acta, 1996, 324(1): 21-27).

### d) Incorporation into membranes and films

U. S. patent No. 2,475,793 (1949) describes immobilized cholinesterase in the form of an insoluble complex with the stroma of mammalian erythrocytes which can be considered as a biological membrane. However, the activity of cholinesterase lowered after several days of storage in a refrigerator which implies that, even after further adjustments, the enzyme was not stable enough.

Patents Nos. CZ284970 (1997) and CZ6566 (1997) cover a platinum electrode coated by a graphite layer of modified cobaltous naphthalocyanate and an enzyme membrane with immobilized cholinesterase. The enzyme membrane was prepared from the mixture of bovine serum albumin, butyrylcholinesterase solution, glutardialdehyde solution as a crosslinking agent, and phosphate buffer in pH 7.4. The membrane was formed by drying the mixture at room temperature.

Patent No. CN101586100 (2012) claims immobilization of cholinesterase on a carrier of water soluble polymers such as polyvinyl alcohol, polyvinyl pyrrolidon, or Arabic gum in phosphate buffer. The carrier also comprises a composite material formed by the water soluble component and lipoplast in the form of a lipophilic polymer. Bovine serum albumin was used as a protective agent. The solution with added enzyme was adhered to a solid material by drip or soakage and then dried to form a film.

Furthermore, cholinesterases can be incorporated into a polyacrylic membrane, covalently bounded onto the surface of nylon tubes, or immobilized in the lipid membranes of liposomes (Adv. Mat. Res., 2013, 726-731: 850-53). Additionally, immobilization of the enzyme in nitrocellulose film was described (Anal. Chim. SSSR, 1990, 45 (7): 1002-1004).

### e) Incorporation into gels

U. S. patent No. 4,241,180 (1980) discloses immobilization of horse serum cholinesterase in a polysaccharide gel based on starch, agar or carrageenan. The gel was created by lyophilization of solidifying gel on filter paper. Immobilized cholinesterase split the substrate forming thiocholine which reduced blue dichloroindophenol into colorless product. When cholinesterase inhibitor was added, no color change was observed. The disadvantage of the polysaccharide gels is their low mechanical strength.

Patent No. DE2946642 (1988) improves the mechanical strength of the gel by using a higher starch concentrations. The starch extrudate was prepared by extrusion of starch paste. The extrudate is consequently dried, ground and sieved.

Patent No. WO8300345 protects the process of binding of the enzyme to the copolymeric gels containing carboxyl groups. The optimal results were achieved when weak acid cation resin Akrilex C-100, prepared by partial hydrolysis of N,N-methylene-bis(acryl amide), was employed as an enzyme carrier. Additionally, immobilization in crosslinked maleinanhydride was also successful.

Patent No. BG50904 (1992) describes immobilization of bovine, calf, and human serum cholinesterase in an agar gel containing the immobilized inhibitor of cholinesterase - galantamine. The enzyme was captured on the sorbent in the medium of low ionic strength. The sorption binding of enzyme was sufficiently strong in such medium, as the ballast protein were washed out with sodium chloride solution. However, the enzyme was released and washed out in the medium with increased ionic strength.

Patent No. WO2009061921 (2009) claims stabilization of cholinesterase by immobilization in the pores of the gels based on natural or synthetic polymers. Moreover, stabilization of cholinesterase in the form of liposomes, nanocomposites, and polyurethane foam was also claimed.

The studies were also aimed at immobilization of cholinesterase into a starch gel using fluorimetry. The starch gel was applied on a polyurethane base (Anal. Chem., 1965, 37(13): 1675-1680) or into the enzyme pads (Anal. Biochem. 1967, 19(3): 587-592).

### f) Incorporation into foams

U. S. patent No. 6,406,876 B1 (2002) discloses a polyurethane foam as a material for immobilization of cholinesterases. This method consists in a reaction between the amino groups of lysine and arginine residues located on the surface of the molecule, while none at the catalytic site gorge. Therefore, binding of the enzyme should not block the entrance of the substrate, inhibitors and reactivators into catalytic site. The preparation of the porous substrate includes the use of prepolymer and surfactants. The result of immobilization depends on the stirring intensity during polymerization. The immobilized enzyme is protected from denaturation, caused by shear forces, using surfactants, e.g. Pluronic C-65 or small amount of glycerol. The immobilized enzyme is stable even at higher temperature and resistant against the different denaturation forces. Other enzymes can be co-immobilized with cholinesterase in the construction of the biosensor.

U.S. patents Nos. 6,759,220 (2004) and 7,422,892 (2008) present variations in the preparation of a polyurethane foam, which influence the characteristics of the foam and the interaction degree between the enzyme and the active site of prepolymer. Butyrylcholinesterase and acetylcholinesterase were immobilized in these patents. The detector containing the enzymes was used for continuous monitoring of inhibitors in air and water.

### g) Crosslinked aggregates

Patent No. RU2182929 (2002) claims crosslinking of glutaraldehyde for immobilization of horse serum cholinesterase. The kit for the determination of cholinesterase inhibitors using such immobilized enzyme was also claimed. Immobilization is carried out by advancing the tape of a fibrous material in an open reactor with a disc stirrer, in which the buffered solution of horse. serum cholinesterase and glutaraldehyde were separately served in two channels.

Patent No. RU2386120 (2010) protects an optical biosensor for the detection of reversible inhibitors containing a fluorescent complex of cholinesterase and the reversible inhibitor lumogen. The complex is immobilized on a neutral substance by enclosing in an N-isopropylacrylamide gel sparsely crosslinked by methylene-bisacrylamide. The crosslinked gel was prepared by photopolymerization on the neutral base.

### h) Immobilization using nanostructures

Carbon nanofibers can be considered as a new type of carrier. The nanofibers were carboxylated and ultrasonically dispersed in a solution. A porous membrane is formed upon solvent removal, which can bind cholinesterase through carbodiimide (J. Mater. Chem. B, 2014, 2(7): 915-922).

### i) Immobilization using covalent bond

Specific binding reaction between the enzyme and the substrate are utilized in this immobilization method. U. S. Patent No. 3,519,538 A (1970) declares silica gel, different silicates (bentonite, wollastonite), and metal oxides (alumina, hydroxy apatite, or nickel oxide) as the sorbents covalently binding the enzyme. The solvent was modified by the reaction with aminopropyl trimethoxysilane in toluene. Primary amino groups were then bonded to the enzyme by known immobilization procedures, e.g. using dicyclohexylcarbodiimide. The prepared derivate was then diazotized and coupled with the enzyme. Amino groups of the modified sorbent can also be activated by the reaction with thiophosgene forming an isothiocyanoalkylsilane derivative. The reaction between the enzyme and glutaraldehyde producing a Schiff base is on of the most frequently used.

The subject of patent No. DE1768934 (1972) is a general method of enzyme immobilization on an insoluble polymeric carrier with a covalently bond reversible inhibitor. The enzyme can be released from the bond by changing the pH and/or ionic strength of the medium, or by competitive displacement.

U. S. patent No. 3,809,616 A (1974) discloses a carrier of filter paper with modified polyacrolein and horse serum cholinesterase. Polyacrolein was sulfited and crosslinked with an aliphatic diamine. Cholinesterase was then bounded either covalently to residual carbonyl groups or ionically to amino groups of modified polyacrolein. The paper was impregnated by the solution of N-methyl indoxyl butyrate in isopropanol and a color change was observed as the indoxyl, created by the hydrolysis of the ester, was oxidized into a deep green product. No color change was observed when cholinesterase inhibitors were present in air.

U. S. patent No. 7,572,764 (2009) claims recombinant human acetylcholinesterase, purified bovine fetal serum acetylcholinesterase, and butyrylcholinesterase conjugated to methoxypolyethylene glycol 5000 or 20000 which was activated by succinimidyl propionate. Comparing to the native enzyme, the conjugates have improved resistance to blood proteases and thus increased stability in the blood circulation. Therefore, they can be used to detoxify the organism from organophosphates. The activation of terminal hydroxyl groups of polyethers by succinimidyl derivatives can be utilized not only for the preparation of the soluble conjugates, but also for the preparation of the enzymes immobilized on the insoluble carriers containing hydroxyl groups. Besides amino groups, thiol groups on cysteine residues are convenient for the attachment of proteins to polyethylene glycol.

The same principle is employed in patent No. WO2009139905 which protects the conjugates of cholinesterase with the insoluble non-peptide polymers with improved properties.

Patent No. EP 1561100 (2005) discloses a specific process for binding of acetylcholinesterase to an aluminum oxide transistor by cyanuric chloride. The ion selective transistor containing the immobilized enzyme forms a biosensor which releases the enzyme upon the action of electric field.

Patent No. WO200806495 (2008) claims immobilization of electric eel acetylcholinesterase using a conjugate with avidin, which was created by glutaraldehyde. The conjugate reacts with biotin bound to the surface of a plastic microtiter plate. The system can be used as a biosensor.

Cholinesterase was also bound to a piezoelectric sensor consisting a quartz crystal coated by a thin gold layer with an attached layer of 11-mercaptoundecanoic acid with cocaine derivative which inhibits the enzyme. The enzyme is released in the presence of other inhibitors (Anal. Bioanal. Chem. 2005; 382(8): 1904-1911).

### j) Immobilization using antibodies

Patent No. EP073889(B1) (2013) protects immobilization of cholinesterase on a fragment of mouse antibodies against human serum cholinesterase, which is adsorbed in the wells of a polystyrene microtiter plate. The complex of the antibody fragment and cholinesterase is used for determining serum cholinesterase activity, which decreases during cirrhosis or liver cancer. The assay is based on the affinity of the biotinylated Aleuria aurantia lectin to cholinesterase. As glycoproteins, cholinesterases interact with lectins.

Patent No. JP2004257996 (A) (2004) claims easily obtainable lentil lectin and a labeled antibody for its assay.

### Detection systems

The above described mechanisms for cholinesterase immobilization form the basic principles for the development of the detection systems which can be applied in the detection of cholinesterase inhibitors in the environment. U.S. patent No. 3,689,224 (1972) protects a laminated sheeted device covered in monochlorotrifluoroethylene or tetrafluoroethylene polymers and aluminum foil. U. S. patent No. 3,809,617A (1974) covers the combined detection discs and patent No. WO8504424 (1985) claims a simple detector kit with two contact surfaces for the detection.

The detection tubes for cholinesterase inhibitors represent an original solution using a lyophilized butyrylcholinesterase filled with gelatin as an enzyme stabilizer (Int. J. Environ. Anal. Chem., 1979, 6(2): 89-94). The solution was protected in patent No. CZ285242 (1993) which claims a detection tube for indicating warfare agents based on cholinesterase inhibitors in air and water.

It can be implied that the detection tubes are one of the progressive means for the cholinesterase inhibitor detection in the environment. Patent No. CZ285242 discloses a detection system containing cholinesterases (acetylcholinesterase and butyrylcholinesterase) immobilized on cellulose granules in size of 0.8-1.2 mm or spherical pellets in size 0.8-1.25 mm prepared by extrusion-spheronization (Čes. slov. farm., 2012; 61(5), 234-239). Both, the granules and the pellets are placed in the glass tubes. However, the granules and the pellets provide only a light discoloration and a rather faint and non-uniform color transition upon enzymatic colorimetric reaction using the chromogenic reagents or substrates.

These drawbacks are eliminated by the present invention.

### Summary of the invention

The present invention pertains to the spherical pellets, particularly designed for the detection of cholinesterase inhibitors by enzymatic hydrolysis of substrate acetylthiocholine and/or butyrylthiocholine producing the corresponding thiol, which is indicated by a color change in a redox indicator or by enzymatic hydrolysis of a chromogenic substrate, and containing the immobilized enzyme based on cholinesterase in activity of 1 to 100 nkat.g⁻¹. In a preferred embodiment, the enzyme is immobilized in a porous, water insoluble inorganic material with specific surface area ranging between 200 and 400 m².g⁻¹, preferably equal to 300 m².g⁻¹; bulk density ranging between 0.05 and 0.25 g.l⁻¹, preferably equal to 0.15 g.l⁻¹; pH of aqueous extract ranging between 6 and 8, preferably equal to 7.4; and mean agglomerated particle size ranging between 50 and 150 µm, preferably between 60 and 120 µm; mixed with at least one spheronizing agent, wherein at least 75% of the resulting pellets have mean particle size between 0.1 and 3.0 mm, preferably between 0.8 and 1.25 mm.

The fraction of the immobilized enzyme based on cholinesterase in a porous, water insoluble inorganic material preferably equals from 0.01 to 1% by wt. of the total weight of the pellets to ensure the activity of the immobilized enzyme based on cholinesterase in the range between 1 and 100 nkat.g⁻¹.

According to an aspect of a preferred embodiment, the enzyme based on cholinesterase is acetylcholinesterase and/or, preferably, butyrylcholinesterase.

According to another aspect of a preferred embodiment, the porous, water insoluble inorganic material consists of magnesium aluminometasilicate.

According to further aspect of a preferred embodiment, at least one spheronizing agent is microcrystalline cellulose.

According to different aspect of a preferred embodiment, the content of porous, water insoluble inorganic material in a mixture with at least one spheronizing agent is equal to 1-90% by wt. preferably 5-50% by wt. of the total weight of the mixture.

The present invention also relates to a method of producing such spherical pellets by extrusion-spheronization or rotogranulation using water or water buffer in the range of pH 6-9 as a wetting agent, wherein residual water content after drying of the pellets does not exceed 7.5% by wt. of the total weight of the pellets.

According to an aspect of a preferred embodiment, the pellets are impregnated by the solution or the dispersion of the enzyme based on cholinesterase in water or water buffer in the range of pH 6-9, wherein residual water content after drying of the pellets does not exceed 7.5% by wt. of the total weight of the pellets.

According to another aspect of preferred embodiment, the pellets are placed in the glass detection tubes in an amount necessary to reach the activity of the immobilized enzyme based on cholinesterase in the range between 0.1 to 10 nkat, preferably 3 nkat, in one detection tube.

The present invention also relates to a use of the above-stated spherical pellets for the detection of cholinesterase inhibitors by enzymatic hydrolysis of substrate acetylthiocholine and/or butyrylthiocholine producing the corresponding thiol which is indicated by a color change in a redox indicator or by method of enzymatic hydrolysis of a chromogenic substrate.

The present invention further pertains to a detection tube for detection of cholinesterase inhibitors by enzymatic hydrolysis of substrate acetylthiocholine and/or butyrylthiocholine producing the corresponding thiol indicated by color change in a redox indicator or by enzymatic hydrolysis of a chromogenic reagent, which is characterized by the content of the above-stated spherical pellets, substrate acetylthiocholine and/or butyrylthiocholine, and a chromogenic reagent.

As stated above, the present invention pertains to the compositions of the spherical pellets containing the immobilized acetylcholinesterase and/or butyrylcholinesterase in activity of 1 to 100 nkat.g⁻¹, which are placed in the glass detection tubes. The enzyme is immobilized in an inorganic sorbent based on magnesium aluminometasilicate by impregnation with the enzyme-containing buffer solution in range of pH 6-9. The pellets are dried after impregnation and placed in the glass tubes. The tubes containing pellets act as the detectors of nerve agents in the environment.

The tube comprises two layers and two glass vials with the solutions. The indicator layer contains pellets with immobilized acetylcholinesterase and/or butyrylcholinesterase. The second layer consists of crushed glass impregnated by substrate acetylthiocholine and/or butyrylthiocholine and the chromogenic reagent (Ellman's reagent). Both vials are filled with a buffer solution in the range of pH 6-9.

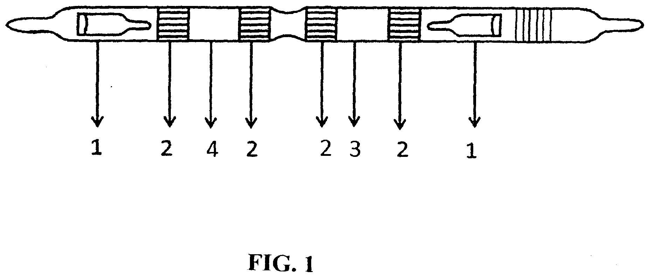

Upon the detection, the vial in the indicator layer is broken by a metal spike and the content is shaken onto the pellets. The equivalent volume of air is drawn into the tube. Afterwards, the second vial is broken and the content is shaken through the crushed glass onto the pellets. A color change in the layer containing the pellets is assessed. If the color changes to yellow, no nerve agents are present in the environment. Otherwise, the color remains unchanged, or the yellow discoloration takes more time. Fig. 1 illustrates one of many possible embodiments of the detection tube, where the reference numeral 1 denotes the buffer-containing vial, the reference numeral 2 denotes the distant sealing elements, the reference numeral 3 denotes the pellet-containing indicator layer, and the reference numeral 4 denotes the layer consisting of crushed glass impregnated by the substrate and the chromogenic reagent.

The detection of nerve agents is based on the enzymatic colorimetric reaction, when cholinesterase hydrolysis of acetylthiocholine or butyryltiocholine to corresponding acid and thiocholine occurs in the absence of nerve agents. Thiocholine further reacts with Ellman's reagent (5,5'-dithiobis-(2-nitrobenzoic acid)) to form 2-nitro-5-thiobenzoic acid (Fig. 2). 2-nitro-5-thiobenzoic acid causes the yellow discoloration of the detector, which is detectable by the naked eye or spectrophotometrically at 412 nm. If no nerve agents are contained in the environment, the tubes changes color. If no color change occurs, the environment is contaminated by nerve agents, as nerve agents inhibit the enzyme which prevents the release of thiocholine and subsequent color reaction.
The reaction scheme of Ellman's reaction:

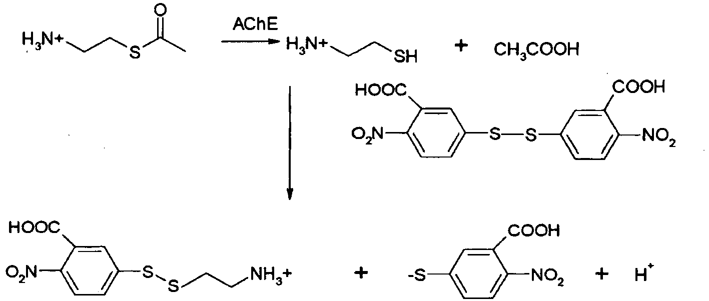

Besides Ellman's reagent, the detection of nerve agents can be carried out using other chromogenic reagents or substrates, e.g. indoxyl acetate, which is hydrolyzed into indoxyl and acetic acid. Afterwards, indoxyl is spontaneously converted into indigo and blue color develops in the solution (Fig. 3). The detection with the tube is similar to the previous process.
The reaction scheme with indoxyl acetate:

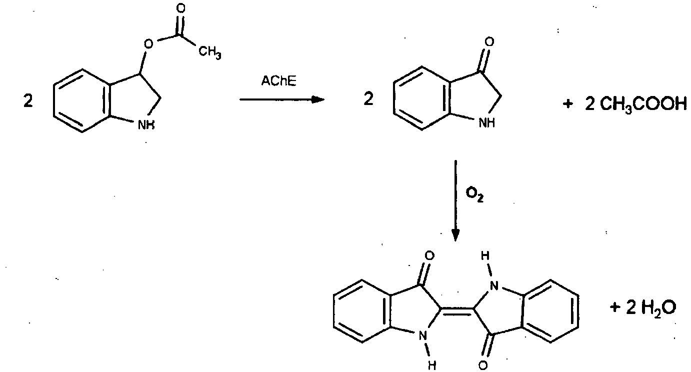

The aforementioned drawbacks can be eliminated in the present invention by using substances which are industrially used as the sorbents for biologically active substances and are based on magnesium aluminometasilicates, preferably, but by no means limited to, in the form of commercially available Neusilin®. Even the addition of the silicates (Čes. slov. farm., 2012; 61(5), 234-239), modifying the prior art, did not lead into the desired effect regarding the different physicochemical properties of these substances. For instance, the discoloration PANTONE 116 U occurs upon use of Ellman's reagent in activity 30 nkat.g⁻¹ of the carrier consisting of Neusilin®, yet much less intense discoloration PANTONE 160 U arises on a carrier consisting of cellulose (according to the PANTONE FORMULA GUIDE).

Neusilin® represents an entirely unique sorbent with quite exceptional sorption properties. It consists of miniature spherical agglomerated particles with an extremely large specific surface area which ranges between 200 and 400 m².g⁻¹ (BET) and low bulk density which ranges between 0.05 and 0.25 g.l⁻¹. pH of the aqueous extract of Neusilin® (5% dispersion) ranges between 6-8, which corresponds to the environment needed for the highest enzymatic activity of cholinesterases. Considering the theory of mixing and further processing into pellets, the spherical character of Neusilin® particles with mean particle diameter ranging between 50 and 150 µm provides a good condition for high uniformity of the final mixture when mixed with powdered substances with similar particle size distribution. This can lead into a better final uniformity of pellet surface discoloration.

Neusilin® US-2 was advantageously used as its specific surface area is 300 m²g⁻¹, bulk density 0.15 g.l⁻¹ and mean particle size 60-120 µm. pH of its 5% aqueous dispersion is 7.4, which represents an extremely suitable environment for the protease enzymatic activity corresponding to the physiological blood pH. This is an indisputable advantage over previously used sorbents of silicates and aluminum oxides, which are not spherical, have not been added in the form of porous agglomerates, and do not have such an advantageous particle size distribution, which therefore leads into poor flow properties in general. Moreover, the aqueous extracts of these sorbents is often not neutral.

Neusilin® is present in the pellets in combination with at least one spheronizing agent, allowing the processing of the mixture into the spherical pellets, e.g. microcrystalline cellulose, preferably, but by no means limited to, in the form of commercially available Avicel® PH 101.

The concentration of inorganic material, preferably Neusilin® US-2, in a mixture with at least one suitable spheronizing agent or a mixture of several materials, preferably Avicel® PH 101, ranges in 1-90% in wt., preferably 5-50% in wt. of the total weight of the mixture.

A suitable ratio of the mean particle size, about 90 µm for Neusilin® US-2 and about 50 µm for Avicel® PH 101, is advantageous to achieve a highly uniform mixture. The pellets themselves are formed by extrusion-spheronization or rotogranulation using water or water buffer in the range of pH 6-9 as a wetting agent, wherein residual water content after drying of the pellets does not exceed 7.5% by wt. of the total weight of the pellets

At least 75% of the resulting pellets have mean particle size between 0.1 and 3.0 mm, preferably 0.8 and 1.25 mm. Comparing to the granules claimed in the patent No. CZ285242, the pellets have the advantage of regular shape and larger surface area in the same particle size distribution, which causes the more uniform surface discoloration.

The resulting pellets are impregnated by the solution or the aqueous dispersion of cholinesterases using water or water buffer in the range of pH 6-9, wherein residual water content after drying does not exceed 1% by wt. The suspension containing 40-1200 µl of stock solution consisting of the enzyme in phosphate buffer in pH 7.6, 5% of dextran, and 0.5% of Slovasol SF-10 is used for impregnation. The pellets are immersed into the mixture for 15 min and subsequently dried.

The immobilized cholinesterases are preferably represented in the present invention in the form of acetylcholinesterase, butyrylcholinesterase, or mixture thereof in an unlimited ratio. Butyrylcholinesterase itself is utilized preferably, as it allows the use of chromogenic substrates, i.e. the substances which are directly hydrolyzed forming a colored product, for instance indoxyl acetate, which can be uncertain in the case of acetylcholinesterase since the hydrolysis is slower and the enzyme is inhibited by the reaction products. The ratio of cholinesterases to the sorption inorganic material ranges in 0.01-1% by wt. of the total weight of the pellets.

The resulting pellets are placed in the glass detection tubes so that the enzyme activity ranges between 0.1 and 10 nkat, preferably 3 nkat, in one detection tube. Comparing to the granules, no dust fraction is created during the pellet manipulation or their loading into the tubes. This is beneficial as the creating of the dust fraction can lead into the loss of the outer layer with adsorbed enzyme and to the more difficult assessment of nerve agents. The regular pellet particles have much better flow properties and occupy a smaller space, which has a positive effect in the pellet manipulation and their loading into the tubes.

The pellets containing the immobilized enzymes are then used for the detection of cholinesterase inhibitors by enzymatic hydrolysis of substrate acetylthiocholine or butyrylthicholine or mixture thereof forming the corresponding thiol, which is indicated by a color change in a redox indicator or by enzymatic hydrolysis of chromogenic substrate.

### Examples

The present invention will now be further illustrated by, but is by no means limited to, the following examples.

### THE EXAMPLES

### Examples 1 to 3

**Tab. 1 Pellet compositions in weight parts**

| *Example* | *1* | *2* | *3* |
|---|---|---|---|
| Lactose monohydrate | 88.83 | 69.09 | 49.35 |
| Neusilin® US-2 | 9.87 | 29.61 | 49.35 |
| Avicel® PH 101 | 81.00 | 81.00 | 81.00 |
| Avicel® RC 581 | 20.30 | 20.30 | 20.30 |
| Buffer in pH 7.4 | 95.70 | 143.55 | 182.70 |
| Kolidon K90 | 14.30 | 21.45 | 27.30 |

### Preparation of the pellets

All examples are processed by the same method.
- Lactose monohydrate, eventually Neusilin® US-2, Avicel® PH 101 and Avicel® RC 581 are mixed during 3 min in a high-speed mixer (Tefal Kaleo, France) until a homogenous mixture is formed.
- Kolidon K90 is dissolved in phosphate buffer in pH 7.4 (according to Ph. Eur.) by constant stirring using an electromagnetic stirrer. The binder solution is thus obtained.
- The binder solution is added into the dry ingredients and the mixture is mixed for 10 min in a high-speed mixer (Tefal Kaleo, France) until a wet dispersion is formed.
- The wet mass is extruded through a 1.25 mm mesh screen at 100 rpm using an extruder (PharmTex 35T, Wyss and Probst Engineering, Switzerland).
- The extrudate is spheronized for 5 min at 1000 rpm using a spheronizer (PharmTex 35T, Wyss and Probst Engineering, Switzerland).
- The resulting pellets are dried at 30 °C for 72 h in a hot air oven (Horo 038A, Dr. Ing. A. Hoffman GmbH, Germany) to a moisture content below 0.5%.

### Evaluation of the pellets

**Tab. 2 Relative frequency of the sieve fractions in % by wt.**

| *Example* | *1* | *2* | *3* |
|---|---|---|---|
| 0 - 1.00 mm | 2.7 | 1.5 | 2.0 |
| 1.00 - 1.25 mm | 30.3 | 15.7 | 20.4 |
| 1.25 - 2,00 mm | 66.5 | 81.9 | 71.5 |
| over 2.00 mm | 0.5 | 1.0 | 6.0 |

**Tab. 3 Examples of impregnation**

| *Example* | *3 a* | *3b* | *3 c* |
|---|---|---|---|
| AChE (nkat.g⁻¹ of the carrier) | 32,25 | - | 20 |
| BuChE (nkat.g⁻¹ of the carrier) | - | 37,5 | 20 |
| Dextran (% by wt.) | 16,125 | 6,25 | - |
| Slovasol (% by wt.) | 1,625 | 6,25 | 10 |

According to example 3, 80 ml of aqueous suspension is used for impregnation of 100 g of pellets.

### Preparation of impregnated pellets

A suspension containing 40-1200 µl of stock solution consisting of the enzyme in phosphate buffer in pH 7.6, 5% of dextran, and 0.5% of Slovasol SF-10 is used for impregnation. A total volume of 80 ml of the suspension is used for impregnation of 100 g of pellets. The pellets are immersed into the mixture in a suitable vessel for 15 min and subsequently dried at 35° C for 24 h.

The tube contains (in the direction of contaminated air flow):
- The vial comprising a buffer solution in a volume of 0.03 ml.
- The layer of the crushed glass impregnated by the substrate and the indicator (possibly only the substrate) in the length of 10 mm and the weight of about 0.1 g.
- The layer of the pellets impregnated by the enzyme in the length of 10 mm and the weight of about 0.05 g.
- The vial comprising a buffer solution in a volume of 0.03 ml.

The layers are fixed and separated from each other by the gas and liquid permeable star-shaped sealing elements made of PE.

### Example 4

Both ampules contain phosphate buffer in pH 8.0.

The crushed glass with grain size of 0.7-0.9 mm is impregnated by the methanol solution comprising 2% of acetylthiocholine iodide and 0.2% of 5,5'-dithiobis-(2-nitrobenzoic acid).

Pellets of examples 1, 2, and 3 are impregnated by acetylcholinesterase in activity of 10 nkat.g⁻¹ of the carrier.

Indication effect - functional enzyme - discoloration, remains white after inhibition.

### Example 5

Both ampules contain phosphate buffer in pH 7.4.

The crushed glass with grain size of 0.7-0.9 mm is impregnated by methanol solution comprising 2.5% of butyrylthiocholine jodide and 0.15% of 2,6-dichlorophenolindophenol.

Pellets of examples 1, 2, and 3 are impregnated by butyrylcholinesterase in activity of 30 nkat.g⁻¹ of the carrier.

Indication effect - functional enzyme - decoloration of blue layer, remains blue after inhibition.

### Example 6

Both ampules contain phosphate buffer in pH 8.2.

The crushed glass with grain size of 0.7-0.9 mm is impregnated by 2% methanol solution of indoxyl acetate.

Pellets of examples 1, 2, and 3 are impregnated by butyrylcholinesterase in activity of 30 nkat.g⁻¹ of the carrier.

Indication effect - functional enzyme - blue discoloration, remains white after inhibition.

### Example 7

Both ampules contain phosphate buffer in pH 7.4.

The crushed glass with grain size of 0.7-0.9 mm is impregnated by methanol solution comprising 1% of acetylthiocholine iodide, 1% of butyrylthiocholine iodide and 0.15% of 2,6-dichlorophenolindophenol.

Pellets of examples 1, 2, and 3 are impregnated by the mixture of acetylcholinesterase and butyrylcholinesterase, both in activity of 20 nkat.g⁻¹ of the carrier.

Indication effect - functional enzyme - decoloration of blue layer, remains blue after inhibition. The general scheme of the detection tube and its contents is shown in Fig. 1.

Tube working procedure
- The bottom vial is crushed and its content is shaken on the layer of pellets containing the enzyme.
- The contaminated air in a volume of 1 1 is sampled manually or by an electric pump.
- Incubation takes effect for 2 min.
- The top vial is crushed and its content is shaken through the crushed glass layer onto the pellet layer to wash off the impregnated reagents.
- The discoloration of the pellet layer is assessed.

If no nerve agents are present in the environment, the system is functioning normally and reduction of the chromogenic reagent or hydrolysis of chromogenic substrate accompanied by a color change (discoloration or decoloration of the indicator layer) takes place. In the presence of the contaminants, the color remains unchanged or the discoloration/decoloration takes more time.

The concentration of the environment contaminants can be determined by the length of the discoloration/decoloration process.

## Claims

1. Spherical pellets designed for the detection of cholinesterase inhibitors by enzymatic hydrolysis of substrate acetylthiocholine and/or butyrylthiocholine producing a corresponding thiol which is indicated by a color change of a redox indicator, or by enzymatic hydrolysis of a chromogenic substrate, and containing an immobilized enzyme based on cholinesterases in the activity of 1 to 100 nkat.g⁻¹, **characterized in that** the enzyme is immobilized in a porous, water insoluble magnesium aluminometasilicate with specific surface area ranging between 200 and 400 m².g⁻¹, preferably equal to 300 m².g⁻¹; bulk density ranging between 0.05 and 0.25 g.l⁻¹, preferably equal to 0.15 g.l⁻¹, pH of aqueous extract ranging between 6 and 8, preferably equal to 7.4; and mean agglomerated particle size ranging between 50 and 150 µm, preferably between 60 and 120 µm; mixed with at least one spheronizing agent, wherein at least 75% of the resulting pellets have mean particle size between 0.1 and 3.0 mm, preferably 0.8 and 1.25 mm

2. Spherical pellets according to claim 1, **characterized in that** the fraction of the immobilized enzyme based on cholinesterase in a porous, water insoluble magnesium aluminometasilicate preferably equals from 0.01 to 1% by wt. of the total weight of the pellets to ensure the activity of the immobilized enzyme based on cholinesterase in the range between 1 and 100 nkat.g⁻¹.

3. Spherical pellets according to the claims 1 and 2, **characterized in that** the enzyme based on cholinesterase is acetylcholinesterase and/or, preferably, butyrylcholinesterase.

4. Spherical pellets according to the claims 1-3, **characterized in that** at least one spheronizing agent is microcrystalline cellulose.

5. Spherical pellets according to the claims 1-4, **characterized in that** the content of porous, water insoluble magnesium aluminometasilicate in a mixture with at least one spheronizing agent is equal to 1-90% by wt. preferably 5-50% by wt. of the total weight of the mixture.

6. Process of manufacturing the spherical pellets according to claim 1-5, **characterized in that** the spherical pellets are prepared by extrusion-spheronization or rotogranulation using water or water buffer in the range of pH 6-9 as a wetting agent, wherein residual water content after drying of the pellets does not exceed 7.5% by wt. of the total weight of the pellets.

7. Process according to claim 6, **characterized in that** the dried pellets are impregnated by the solution or the dispersion of the enzyme based on cholinesterase in water or water buffer in the range of pH 6-9, wherein residual water content after drying of the pellets does not exceed 7.5% by wt. of the total weight of the pellets.

8. Process according to claim 7, **characterized in that** the pellets are placed in the glass detection tubes in an amount necessary to reach the activity of the immobilized enzyme based on cholinesterase in the range between 0.1 to 10 nkat, preferably 3 nkat, in one detection tube.

9. Usage of the spherical pellets according to claims 1-5 for detection of cholinesterase inhibitors by enzymatic hydrolysis of substrate acetylhiocholine and/or butyrylthiocholine producing the corresponding thiol which is indicated by a color change in a redox indicator or by method of enzymatic hydrolysis of a chromogenic substrate.

10. Detection tube for the detection of cholinesterase inhibitors by enzymatic hydrolysis of substrate acetylthiocholine and/or butyrylthiocholine producing the corresponding thiol indicated by a color change in a redox indicator or by enzymatic hydrolysis of a chromogenic reagent, **characterized in that** the content consists of the spherical pellets according to claims 1-5, substrate acetylthiocholine and/or butyrylthiocholine, and a chromogenic reagent.

## Patentansprüche

1. Kugelförmige Pellets, die für den Nachweis von Cholinesteraseinhibitoren durch enzymatische Hydrolyse eines Acetylthiocholin- und/oder Butyrylthiocholinsubstrats vorgesehen sind, was ein entsprechendes Thiol erzeugt, was durch eine Farbänderung eines Redoxindikators oder durch eine enzymatische Hydrolyse eines chromogenen Substrats angezeigt wird, und enthaltend ein immobilisiertes Enzym auf Grundlage von Cholinesterasen in der Aktivität von 1 bis 100 nkat/g, **dadurch gekennzeichnet, dass** das Enzym in einem porösen, wasserunlöslichen Magnesiumaluminometasilikat mit einer spezifischen Oberfläche im Bereich zwischen 200 und 400 m²/g, vorzugsweise gleich 300 m²/g immobilisiert ist; eine Schüttdichte zwischen 0,05 und 0,25 g/l beträgt, vorzugsweise gleich 0,15 g/l ist; ein pH-Wert des wässrigen Extrakts zwischen 6 und 8 beträgt, vorzugsweise gleich 7,4 ist; und eine mittlere agglomerierte Partikelgröße zwischen 50 und 150 µm beträgt, vorzugsweise zwischen 60 und 120 µm; vermischt mit mindestens einem Sphäronisierungsmittel, wobei mindestens 75 % der resultierenden Pellets eine mittlere Partikelgröße zwischen 0,1 und 3,0 mm, vorzugsweise 0,8 und 1,25 mm aufweisen.

2. Kugelförmige Pellets nach Anspruch 1, **dadurch gekennzeichnet, dass** der Anteil des immobilisierten Enzyms auf Cholinesterasebasis in einem porösen, wasserunlöslichen Magnesiumaluminometasilikat vorzugsweise zwischen 0,01 bis 1 Gew.-% des Gesamtgewichts der Pellets gleicht, um sicherzustellen, dass die Aktivität des immobilisierten Enzyms auf Cholinesterasebasis im Bereich zwischen 1 und 100 nkat/g liegt.

3. Kugelförmige Pellets nach den Ansprüchen 1 und 2, **dadurch gekennzeichnet, dass** das Enzym auf Cholinesterasebasis Acetylcholinesterase und/oder vorzugsweise Butyrylcholinesterase ist.

4. Kugelförmige Pellets nach den Ansprüchen 1-3, **dadurch gekennzeichnet, dass** mindestens ein Sphäronisierungsmittel mikrokristalline Cellulose ist.

5. Kugelförmige Pellets nach den Ansprüchen 1-4, **dadurch gekennzeichnet, dass** der Gehalt von porösem, wasserunlöslichem Magnesiumaluminometasilikat in einer Mischung mit mindestens einem Sphäronisierungsmittel gleich 1-90 Gew.-%, vorzugsweise 5-50 Gew.-% des Gesamtgewichts der Mischung beträgt.

6. Verfahren zum Herstellen der kugelförmigen Pellets nach den Ansprüchen 1-5, **dadurch gekennzeichnet, dass** die kugelförmigen Pellets durch Extrusions-Sphäronisation oder Rotogranulation unter Verwendung von Wasser oder Wasserpuffer im Bereich eines pH-Werts von 6-9 als ein Benetzungsmittel angefertigt werden, wobei ein Restwassergehalt nach einem Trocknen der Pellets 7,5 Gew.-% des Gesamtgewichts der Pellets nicht überschreitet.

7. Verfahren nach Anspruch 6, **dadurch gekennzeichnet, dass** die getrockneten Pellets durch die Lösung oder die Dispergierung des Enzyms auf Cholinesterasebasis in Wasser oder Wasserpuffer im Bereich von pH 6-9 imprägniert sind, wobei ein Restwassergehalt nach einem Trocknen der Pellets 7,5 Gew.-% des Gesamtgewichts der Pellets nicht überschreitet.

8. Verfahren nach Anspruch 7, **dadurch gekennzeichnet, dass** die Pellets in die Glasnachweisröhrchen in einer Menge platziert werden, die erforderlich ist, um eine Aktivität des immobilisierten Enzyms auf Cholinesterasebasis im Bereich von 0,1 bis 10 nkat, vorzugsweise 3 nkat, in einem Nachweisröhrchen zu erzielen.

9. Verwendung der kugelförmigen Pellets nach den Ansprüchen 1-5 zum Nachweis von Cholinesteraseinhibitoren durch enzymatische Hydrolyse eines Acetylthiocholin- und/oder Butyrylthiocholinsubstrats, wodurch das entsprechende Thiol erzeugt wird, was durch eine Farbänderung eines Redoxindikators oder durch eine enzymatische Hydrolyse eines chromogenen Substrats angezeigt wird.

10. Nachweisröhrchen zum Nachweis von Cholinesteraseinhibitoren durch enzymatische Hydrolyse eines Acetylthiocholin- und/oder Butyrylthiocholinsubstrats, was das entsprechende Thiol erzeugt, was durch eine Farbänderung eines Redoxindikators oder durch eine enzymatische Hydrolyse eines chromogenen Substrats angezeigt wird, **dadurch gekennzeichnet, dass** der Inhalt aus den kugelförmigen Pellets nach den Ansprüchen 1-5, einem Acetylthiocholin- und/oder Butyrylthiocholinsubstrat und einem chromogenen Reagens besteht.

## Revendications

1. Pastilles sphériques conçues pour la détection d'inhibiteurs de cholinestérase par hydrolyse enzymatique de substrat acétylthiocholine et / ou butyrylthiocholine produisant un thiol correspondant qui est indiqué par un changement de couleur d'un indicateur redox, ou par hydrolyse enzymatique d'un substrat chromogène, et contenant une enzyme immobilisée à base de cholinestérases dans l'activité de 1 à 100 nkat.g⁻¹, **caractérisées par le fait que** l'enzyme est immobilisée dans un aluminométasilicate de magnésium insoluble dans l'eau, poreux, ayant une surface spécifique se situant dans la plage entre 200 et 400 m².g⁻¹, de préférence égale à 300 m².g⁻¹ ; une masse volumique apparente se situant dans la plage entre 0,05 et 0,25 g.l⁻¹, de préférence égale à 0,15 g.l⁻¹ ; un pH d'extrait aqueux se situant dans la plage entre 6 et 8, de préférence égal à 7,4 ; et une taille moyenne de particules agglomérées se situant dans la plage entre 50 et 150 µm, de préférence entre 60 et 120 µm ; mélangé avec au moins un agent de sphéronisation, au moins 75 % des pastilles résultantes ayant une taille moyenne de particule entre 0,1 et 3,0 mm, de préférence entre 0,8 et 1,25 mm.

2. Pastilles sphériques selon la revendication 1, **caractérisées par le fait que** la fraction de l'enzyme immobilisée à base de cholinestérase dans un aluminométasilicate de magnésium insoluble dans l'eau, poreux, est de préférence égale à 0,01 à 1 % en poids du poids total des pastilles pour assurer l'activité de l'enzyme immobilisée à base de cholinestérase dans la plage entre 1 et 100 nkat.g⁻¹.

3. Pastilles sphériques selon l'une des revendications 1 et 2, **caractérisées par le fait que** l'enzyme à base de cholinestérase est l'acétylcholinestérase et / ou, de préférence, la butyrylcholinestérase.

4. Pastilles sphériques selon l'une quelconque des revendications 1 à 3, **caractérisées par le fait qu'**au moins un agent de sphéronisation est la cellulose microcristalline.

5. Pastilles sphériques selon l'une quelconque des revendications 1 à 4, **caractérisées par le fait que** la teneur en aluminométasilicate de magnésium insoluble dans l'eau, poreux, dans un mélange avec au moins un agent de sphéronisation est égale à 1 à 90 % en poids, de préférence égale à 5 à 50 % en poids du poids total du mélange.

6. Procédé de fabrication des pastilles sphériques selon l'une quelconque des revendications 1 à 5, **caractérisé par le fait que** les pastilles sphériques sont préparées par extrusion-sphéronisation ou rotogranulation à l'aide d'eau ou d'un tampon aqueux dans la plage de pH de 6 à 9 en tant qu'agent mouillant, la teneur en eau résiduelle après séchage des pastilles ne dépassant pas 7,5 % en poids du poids total des pastilles.

7. Procédé selon la revendication 6, **caractérisé par le fait que** les pastilles séchées sont imprégnées par la solution ou la dispersion de l'enzyme à base de cholinestérase dans l'eau ou dans un tampon aqueux dans la plage de pH de 6 à 9, la teneur en eau résiduelle après séchage des pastilles ne dépassant pas 7,5 % en poids du poids total des pastilles.

8. Procédé selon la revendication 7, **caractérisé par le fait que** les pastilles sont placées dans des tubes de détection en verre dans une quantité nécessaire pour atteindre l'activité de l'enzyme immobilisée à base de cholinestérase dans la plage entre 0,1 à 10 nkat, de préférence de 3 nkat, dans un tube de détection.

9. Utilisation des pastilles sphériques selon l'une des revendications 1 à 5 pour la détection d'inhibiteurs de cholinestérase par hydrolyse enzymatique de substrat acétylthiocholine et/ou butyrylthiocholine produisant le thiol correspondant qui est indiqué par un changement de couleur dans un indicateur redox ou par une méthode d'hydrolyse enzymatique d'un substrat chromogène.

10. Tube de détection pour la détection d'inhibiteurs de cholinestérase par hydrolyse enzymatique de substrat acétylthiocholine et / ou butyrylthiocholine produisant le thiol correspondant indiqué par un changement de couleur dans un indicateur redox ou par hydrolyse enzymatique d'un réactif chromogène, **caractérisé par le fait que** le contenu consiste en les pastilles sphériques selon l'une des revendications 1 à 5, le substrat acétylthiocholine et / ou butyrylthiocholine et un réactif chromogène.
